# EUROPEAN PATENT SPECIFICATION

(11) **EP 3 240 842 B1**
(45) Date of publication and mention of the grant of the patent: **03.05.2023**
(21) Application number: 15875380.6
(22) Date of filing: 30.12.2015
(51) Int. Cl.: C09D 129/04, A61K 31/00, A61K 9/28

(54) **NOVEL FILM COATING COMPOSITION**
NEUARTIGE FILMBESCHICHTUNGSZUSAMMENSETZUNG
NOUVELLE COMPOSITION DE REVÊTEMENT DE TYPE FILM

(30) Priority: 01.01.2015 IN 3828MU2014
(43) Date of publication of application: 08.11.2017
(73) Proprietor: Ideal Cures Pvt. Ltd., Goregaon (East) Mumbai 400 063 (IN)
(72) Inventor: PAREEK, Suresh Rukmanand, Goregaon (East) Mumbai 400 063 (IN); NEGI, Sanjay, Goregaon (East) Mumbai 400 063 (IN)
(74) Representative: ZBM Patents - Zea, Barlocci & Markvardsen
(86) International application number: PCT/IN2015/050222
(87) International publication number: WO 2016/108250

(56) References cited:
- EP-A1- 3 106 152
- WO-A1-2007/063553
- WO-A1-2010/052727
- WO-A2-2006/111981
- AU-A1- 2005 251 793
- US-A- 5 595 762
- US-A1- 2009 054 533

## Description

### Field of the Invention

The present invention relates to novel film coating composition having low viscosity at high solids content with maximum spraying rate. The film coating composition exhibits good adhesion and imparts a good smooth surface to the coated substrates. The present invention relates to the field of coating compositions for substrates like pharmaceutical tablets, nutritional supplements, food products, and the like.

### Background of the invention

Film coating is a process in which the substrate such as a tablet or a pellet or a confectionary or a seed is enveloped by a thin layer of polymeric film forming material. Film coating may be carried out for a wide variety of reasons such as changing the color for aesthetic purposes or branding; to protect the material from degradation due to environmental conditions such as moisture, light; to modify the release of the active ingredient of a pharmaceutical composition and such other purposes.

Vinyl polymers are a group of polymers derived from vinyl monomers. Their backbone is an extended alkane chain, made by polymerizing an alkene group (C=C) into a chain (-C-C-C-C-). Polyvinyl alcohol or PVA is the commonly used vinyl polymer, which is water soluble and synthetic in nature represented by chemical formula (C₂H₄O)ₙ. It is commercially manufactured by hydrolysis of polyvinyl acetate. Polyvinyl alcohol is generally considered a nontoxic and non irritant material.

The physical characteristics and specific functional uses of PVA depend on the degree of polymerization and the degree of hydrolysis. Polyvinyl alcohol is classified into two classes namely: partially hydrolyzed and fully hydrolyzed. It is well established in prior art that partially hydrolyzed PVA is used as coating material.

Polyvinyl alcohol is primarily used as a film coating polymer. Other uses of polyvinyl alcohol include use as lubricant, as stabilizing agent for emulsions and viscosity increasing agent for ophthalmic products.

Certain film coating compositions with poly vinyl alcohol have been reported in prior art for instance,
EP0600775 describes in its specification that active ingredients in the formulation are stabilized by a coating comprising a film coating material like poly vinyl pyrrolidone or polyvinyl alcohol and a pore forming agent like microcrystalline lactose or polyethylene glycol etc. No disclosure as to the solids content or the viscosity of polyvinyl alcohol solution.

US 5244429 discloses coatings based on polyvinyl alcohol that improve the buoyant life of elastomeric balloons comprising a combination of polyvinyl alcohol and a carbohydrate extender.US'429 discloses that the viscosity of polyvinyl alcohol may be reduced by addition of hydrogen peroxide, however no disclosure as to the solids content at which the dispersion is made.

WO 1996/01874 discloses a moisture barrier film coating composition which comprises polyvinyl alcohol, soya lecithin, an optional flow aid, an optional colorant, and an optional suspending agent mixed into water.WO'874 discloses dispersing 220 grams of powder in 780 grams of purified water, which amounts to 22% w/w of solids content. No mention of viscosity of the compositions is provided.

Ideal Cure's application WO 2006111980 and subsequent Indian patent IN198511 discloses the use of self emulsifying glyceryl monostearate (SE-GMS) as an anti-tack agent in immediate release film coating compositions. It discloses coating dispersions with solids content of 10-30 %w/w. No mention as to the viscosity of the compositions is provided.

WO 2006111981 and subsequent Indian patent IN198023, filed by Ideal Cures relates to a dry moisture barrier coating comprising polyvinyl alcohol in combination with self-emulsifying glyceryl monostearate, which enhances the desirable coating characteristics of the film coating. The coating contains a flavoring agent, sweetening agent, flow aid, colorant and a plasticizing agent. No mention as to the viscosity of the compositions or the solids content is provided.

WO 2010/132205 discloses film coating systems comprising polyvinyl alcohol, fine particle size detackifiers, a plasticizer- preferably polyethylene glycol or triethyl citrate, a glidant like talc and other pharmaceutical excipients. Another application WO 2010/132204 discloses a moisture barrier coating comprising polyvinyl alcohol, a polymer with pH dependent solubility, and optionally a plasticizer, glidant, alkalizing agent, detackifiers, etc. However, in both these prior art the compositions disclosed had been reconstituted with maximum of 25% solids content and there was no disclosure of viscosity of these compositions.

Ideal cure's application WO2012120364 and WO2012120366 discloses the use of polyvinyl alcohol in film coating compositions. WO 2012/120364 describes a film coating composition comprising a polymer composite base and pharmaceutically acceptable excipients. The composite base comprises polyvinyl alcohol, glyceryl monostearate, polysorbate-80 and diethyl phthalate. But the viscosity of compositions described therein is still high.

WO2010052727 discloses dry film coating compositions comprising a low viscosity cellulose polymer, modified starch and other commonly used coating components.

WO2007063553 discloses dry film coating compositions comprising sodium alginate and a plasticizer. In its background section, it discloses a coating composition comprising dextrose and an auxiliary film-former, for example PVA.

US2009054533 discloses dry film coating compositions comprising tribasic calcium phosphate along with other commonly used compounds.

AU2005251793 discloses coating composition comprising water and a solid component that comprises a sugar, a binder, hydroxyalkyl cellulose, a water soluble polymer, optionally, a therapeutic agent, optionally, a second polymer that is water soluble or water dispersible and, optionally, at least one plasticizer.

US5595762 discloses a coating composition comprising at least one film-forming agent, and at least one pore-forming agent. The coating composition also comprises plasticizers and antiadhesive agents.

EP3106152 discloses an orally disintegrating tablet, characterized in that the tablet is coated with a film coating composition comprising: a water-soluble and alcohol-insoluble film coating base; and at least one plasticizer selected from the group consisting of propylene glycol and polyethylene glycol in a liquid state at room temperature; and optionally at least one pharmaceutically acceptable additive.

Spraying highly viscous coating compositions of polyvinyl alcohol at high solids content is very difficult and it may lead to coating issues like gun blockages, tablet sticking etc. Less viscous polyvinyl alcohol based formulations help in providing good spray ability for the coating preparations. It is further seen that none of the prior arts provide a solution to reduce the viscosity of polyvinyl alcohol while still maintaining its dispersability at high solids content.

Coating materials based on vinyl polymers are known in prior art, especially the ones based on polyvinyl alcohol. However due to inherent properties of stickiness of polyvinyl alcohol it is difficult to achieve coating compositions with higher solids content of polyvinyl alcohol and yet achieve maximum spray rates. Therefore there has been a long felt need for coatings based on polyvinyl alcohol having low viscosity which will reduce coating time and where high spray rates can be achieved.

Over the years, many changes have occurred in the coating material, process and equipment. Earlier tablet coating was carried in small and medium scale batch process, where coating machines (solid and perforated) had a maximum pan diameter of 6" to 60". Over time, high volume batch coaters were developed with 66"to72" pan diameters which were all equipped with 5 to 9 spray guns for spraying coating material. More recently new generation continuous coating machines have been introduced where coating can be carried out in a continuous process. Continuous coaters are horizontal tunnels with 10" to 30" diameters, where the numbers of guns for spraying coating materials are 22 to 28. In these continuous coating processes the solvents must dry at the faster rate to allow continuous output of tablets. The compositions of the present invention use less water for dispersion, which dry at a faster rate after coating leading to higher production outputs and help in making the process efficient by saving time and energy and help in reducing carbon footprints.

In both these coating operations i.e., batch coating as well as continuous coating, it is necessary that equilibrium between the rate of application of coating material and rate of evaporation of water (as a solvent) is maintained. Slight deviation from the same may cause disturbance of equilibrium and result in coating problems. Use of viscous coating material during coating operations of larger batch size when carried out in coating machines with multiple guns has numerous disadvantages, including difficulty in spraying, tablet sticking, peeling of film, gun blockages etc. Thus it is extremely important that these guns don't block because of highly viscous material being sprayed through gun nozzle. There is therefore a strong need for coating compositions having much lower viscosity at high solids content, which provide high sprayability and easy usability in high volume batch coating and continuous coating operations without causing blocking of guns. It is also desirable that these coatings provide good film adhesion and smooth finish to the coated substrates.

Hence, there is a need to have a coating composition that increases the industrial compliance of vinyl polymer and reduces its process related flaws. There is a further need for coating compositions which are easily dispersible and easily sprayable at high solids content, whereby they use lower solvents i.e. water, which consume less energy, thereby helping in reduction of carbon footprint.

### Object of the invention

An object of the invention is to provide a novel film coating composition that provides higher production outputs, has lower viscosity which can be sprayed effectively at high solids content using less solvent i.e. water. This unique character of the present composition in one embodiment makes it ideal for high volume batch process and continuous coating applications.

It is further an object of the invention to provide coating composition that has good film adhesion, imparts smooth surface to the substrates and reduces carbon footprints.

### Summary of Invention

The present invention describes novel film coating composition of poly vinyl alcohol (PVA) in combination with sugar and a plasticizer or a combination of plasticizers resulting in a composition giving high solids content where higher spray rate can be achieved with lower solvent, that help in reduction of coating time and provide smooth coating, which is non tacky and disperses easily in water at ambient temperature. Further the resultant film exhibits good adhesion and imparts smooth surface to the substrate and helps reduce carbon footprints.

### Detailed description of the invention

Definitions:
By "Polymer" as used herein refers to the vinyl polymers and their derivative like poly vinyl alcohol.
By "sugar" as used herein mean compounds having single or plurality of saccharide units, preferably the sugar being disaccharides like lactose, lactose mono hydrate or derivatives thereof.
By "waxy" as used herein refers to the substance having hydrophobic activity, further in some instances waxy component is fatty acid derivatives and combination thereof.
The term "plasticizer" as used herein refers to the group of compounds having plasticizing property and helps in forming film with polymers. The plasticizer may be used alone or in combination with other plasticizer.
The term "glidant" as used herein refers to the group of compounds having property of increasing flow of dry powders.
By "additional polymer" as used herein refers to the polymer synthetic or natural in origin having two or plurality of sub units repeated in organized or specific manner.
The term "colorants/pigments" used herein refers to natural and synthetic colorants/pigments including but not limited to red, yellow and black iron oxides, titanium dioxide, calcium carbonate, zinc oxide, FD& C colors, lake colors and mica based pearlescent pigments.

The composition of the present invention concerns to reduce the inherent flaws of polymer, for example coagulation, tackiness, clump formation, high viscosity, non-conformity of dispersion, film unevenness etc.

The film coating composition of the present invention comprising poly vinyl alcohol, a sugar, a wetting agent, a waxy component, a plasticizer, optionally an additional polymer, a glidant, colorants, a flavourant and other pharmaceutically acceptable excipients and may be used in coating pharmaceutical tablet, nutritional supplement, food, confectionery forms, agricultural seed, and the like.

In an embodiment, the present invention provides a dry or liquid coating composition comprising polyvinyl alcohol, a sugar and optionally a wetting agent, a waxy component, a plasticizer, an additional polymer" a glidant, colorants, a flavourant, artificial sweetener and combination thereof.

The present invention also provides film coating compositions for immediate release or modified release of active ingredients.

It is surprising found that combination of sugar and poly vinyl alcohol causes significant reduction in the viscosity of the coating solution. The polymer and sugar in ratio range between 0.5:1 to 3:1 is found to reduce viscosity significantly, even when the coating composition has a solids content of 20 to 45%w/w.

The polymer used in the composition is poly vinyl alcohol (PVA).

The amount of poly vinyl alcohol used in the composition is present in the range of 20 % w/w to 50 % w/w, more preferably 24 % w/w to 42% w/w.

The composition of the present invention includes a sugar selected from group comprising monosaccharide, disaccharide and polysaccharide, more preferably disaccharide.

The monosaccharide sugar may be selected from glucose, galactose, mannose and fructose. The disaccharide sugar may be selected from a group comprising such as sucrose, lactose, lactulose, maltose, trehalose, isomaltose, lactose mono hydrate or derivatives thereof.

The amount of sugar present in the composition is in the range of 5% w/w to 40% w/w, more preferably in the range between 8 % w/w to 35 % w/w.

The composition of the present invention may also include a plasticizer. The plasticizer may be selected from the group comprising phthalate ester, phosphate ester, other ester like citrate, stearate, sebacate, oleate, adipate, etc. oil, glycerol, glycol etc, preferably the plasticizer is ethyl phthalate, methyl phthalate, dipropyl phthalate, diethyl phthalate, glycerine, polyethylene glycol (PEG), triethyl citrate (TEC), di-butyl sebacate (DBS), di-ethyl phthalate (DEP), di-butyl phthalate(DBP), triacetin, more preferably the plasticizer is diethyl phthalate, triethyl citrate and dibutyl phthalate alone or in combination thereof. The plasticizer may also be used in combination with other plasticizer.

The amount of plasticizer used in the composition is present in the range of 5% w/w to 20% w/w, more preferably in the range of 9 % w/w to 16 % w/w.

The composition of the present invention may include a waxy component or a combination of waxy components that are selected from groups comprising fatty acids, glyceryl mono-stearate, glyceryl behenate, glyceryl palmitostearate, magnesium stearate, sorbitan esters, stearic acid, palmitic acid, polyoxyethylene alkyl ethers, lauroyl poly oxyl glycerides and stearoyl polyoxyl glycerides, cetostearyl alcohol, cetyl alcohol, cerosin, propylene glycol monostearate, sorbitan tristearate, sodium stearyl fumarate, stearyl alcohol, hydrogenated vegetable oil, carnauba wax, microcrystalline wax, Dioctyl sodium sulfosuccinate, ethylene glycol stearates, glyceryl monooleate, lanolin, myristic acid, petrolatum/lanolin alcohols, derivatives of stearate.

The amount of waxy component used in the composition may be in the range of 0.1% w/w to 10 % w/w, more preferably in the range of 1% w/w to 7% w/w.

The wetting agent used in the composition of the present invention may be selected from the group comprising ammonium lauryl sulfate, sodium lauryl sulfate, polysorbate-20, polysorbate-40, polysorbate-60, polysorbate-80, more preferably polysorbate-80.

The amount of wetting agent used in the composition may be in the range of 0.1% w/w to 4% w/w more preferably about0.5% w/w to 3% w/w.

The composition of the present invention may additionally comprise other ingredients such as an additional polymer, colorants, a glidant, a flavoring agent and/or a sweetening agent etc.

The composition of the present invention may include additional polymer. The additional polymer may be selected from the group comprising cellulosic polymers like hypromellose (hydroxyl propyl methyl cellulose), hydroxyl propyl cellulose, sodium carboxy methyl cellulose, acrylic polymers, synthetic polymers, methacrylic acid copolymers and derivatives like Eudragit L100, Eudragit L30D, Kollicoat MAE 30 DP and Eudragit L100-55; partially-neutralized poly (methacrylic acid, ethyl acrylate) 1:1 like Kollicoat MAE-100P; ECOPOL L30 D; ECOPOL L100 55; poly(methacrylic acid, methyl methacrylate) 1:2 like Eudragit S, poly vinyl acetate phthalate (PVAP), PVAP - titanium dioxide (PVAP-T), cellulose acetate phthalate (CAP), hydroxyl propyl methyl cellulose acetate succinate (HPMC-AS) and hydroxyl propyl methyl cellulose phthalate (HPMC-P), poly(butyl methacrylate, 2-dimethylaminoethyl methacrylate, methyl methacrylate) 1:2:1 like Eudragit E, and pre-formulated products brands like Acryl-EZE, Sureteric and Chromateric.

The amount of additional polymer in the present invention is in the range of 0.1 % w/w to 30 % w/w, more preferably between 10 % w/w to 25 % w/w.

The glidant used in the composition of the present invention may be selected from group comprising magnesium stearate, Aerosil^{®} (colloidal silicon dioxide), starch and talc, Hydrophobic colloidal silica, magnesium oxide, magnesium silicate, magnesium trisilicate, more preferably talc, present in the amount ranging from 0.1% w/w to 10% w/w, more preferably 2% w/w to 7% w/w.

The colorants/pigments used in the composition of the present invention may be selected from group comprising natural and synthetic colorants/pigments including but not limited tored, yellow and black iron oxides, titanium dioxide, calcium carbonate, zinc oxide, FD& C colors, lake colors, mica based pearlescent pigments and the like.

The amount of colorants in the present invention is in the range of 18 % w/w to 28 % w/w, more preferably between 20 % w/w to 25 % w/w.

In one embodiment the composition of the present invention may be prepared by combining the sugar and plasticizer by any one of the methods comprising heating, stirring, mixing and the like to obtain homogenous dispersion. Poly vinyl alcohol is added gradually to prepare the dry composition of the present invention. Colorants and other pharmaceutical acceptable excipients may be added later on to obtain alternate compositions.

In other embodiment, the composition of the present invention may be prepared by a process comprising the steps of:
i. combining plasticizer with the waxy component, wetting agent and sugar to obtain a homogenous dispersion,
ii. adding poly vinyl alcohol to dispersion of step (i) to obtain dry powder
iii. optionally adding an additional polymer and pharmaceutical acceptable excipients to the dry powder of step (ii) to obtain the composition of the present invention.

The process of preparing the composition of the present invention includes combining the plasticizer, waxy component, wetting agent, sugar and polymer. The combination of these may be achieved by several processes selected from the group comprising heating, stirring, mixing and the like to obtain homogenous dispersion.

After initial mixing, other pharmaceutical acceptable excipients like glidant, an additional polymer, colorants and pharmaceutical excipients may be added to obtain the composition of the present invention.

The film coating composition of the present invention has good adhesion properties and film forming properties.

The film coating may be applied by spray coating process commonly used to coat orally ingestible substrates. The amount of coating applied will depend upon several factors, including the nature and functionality of the film coating, the substrate to be coated and the apparatus employed to apply the coating, etc.

The film coating compositions of the present invention may also be suited for high volume batch process and continuous coating operations. In both these coating operations it is necessary that equilibrium between the rate of application of coating material and rate of evaporation of solvents is maintained. Slight deviation from this equilibrium may result in coating problems. Viscous coating material has numerous disadvantages, including difficulty in spraying, tablet sticking, gun blockages etc. Moreover these coaters have multiple spraying guns which spray coating material onto the tablets. Viscous material may cause accumulation resulting in blockage of spray guns. Thus, it is extremely important that the coating compositions used in such operations have reasonable viscosity at high solids content to provide easy application and efficient solvent evaporation. It is surprisingly seen that combination of polyvinyl alcohol with sugar results in coating compositions having lower viscosity even when they are reconstituted with a solids content of 30-40%, which aid in their easy application during high volume batch process and continuous coating operations. Moreover since the compositions are reconstituted with high solids content they use less amount of solvent, hence its evaporation is also quick and efficient.

Further, since the compositions are reconstituted with high solids content they use less quantity of solvents and thus there is less consumption of energy during their evaporation. In this way the coating compositions also help in reduction of carbon foot prints and make the process energy efficient.

These properties of the present invention such as reconstitution with high solids content coupled with low viscosity and high spraying rates, make it ideal for applications in high volume continuous coating operations.

A non-limiting list of suitable substrates that can be coated with the inventive coating system include compressed tablets, caplets, capsules, cores including pharmaceuticals, nutraceuticals and dietary supplements as well as any other art-recognized orally ingestible core.

Suitable pharmaceutical components that may be coated with the composition of the present invention include, but are not limited to: adrenergic blocking agent; acetyl-cholinesterase inhibitor; analgesic or antipyretics; angiotensin modulator; anthelmintic agents; anti-anxiety agent; antibacterial; antibiotic; anticoagulant; anticonvulsant; antidepressant; antifungal; antihistamine; antimalarial; antimicrobial agent; antipsychotic agent; Antiviral agents; blood glucose lowering drug; calcium channel modulator; diuretic; erectile dysfunction; gastric acid secretion inhibitor; histamine H2-receptor antagonist; inhibitor of steroid Type II 5[alpha]-reductase including; lipid regulating agents; selective Hl- receptor antagonist; vasodilator; vitamins.

Without being limited by the theory, the present invention provides a novel film coating composition comprising poly vinyl alcohol, a sugar, a plasticizer and optionally an additional polymer and other pharmaceutically acceptable excipients. The optimal use of various components of the present composition renders the composition synergistic. The poly vinyl alcohol and sugar in ranges ratio between 0.5: 1 to 3:1 removes most of the processing flaws to industrially un-noticeable units. The film coating composition of the present invention, solids content of 20 to 40% can be achieved.

### ADVANTAGES OF THE INVENTION

*1*. The film coating composition of the present invention has high solids content with low viscosity.
*2*. The film coating composition of the present invention has less tackiness.
*3*. The composition has good adhesion and imparts smooth surface to the substrates.
*4*. The composition of present invention further provides reconstitution at high solids content of 35% solids content, which means it utilizes less solvent for forming dispersion, thereby contributing actively to the reduction in carbon footprints.
*5*. The low viscosity of present coating composition help in the achieving easy manageable coating process with reduced gun choking and makes it ideal for use in high volume batch process and continuous coating operations.

The invention is described in detail herein below with respect to the following examples, which are provided merely for illustration and are not intended to restrict scope of invention in any manner. Any embodiments that may be apparent to a person skilled in the art are deemed to fall within the scope of present invention.

### EXAMPLES

### Example 1: Illustrative composition of the present invention

**Table A: Composition of the present invention**

| **Ingredients** | **Std. Formula as % w/w** |
|---|---|
| Polyvinyl Alcohol | 25 |
| Glyceryl Monostearate | 3 |
| Polysorbate 80 | 1 |
| Triacetin | 12 |
| Lactose Monohydrate | 31 |
| Talc | 5 |
| Colorants | 23 |
| **Total** | **100** |

Process of preparation of composition comprises mixing of 12% of triacetin, 3% of glyceryl monostearate, 1% of polysorbate-80 and 31% of lactose monohydrate to obtain homogenous dispersion. The homogenization of dispersion may be achieved by heating, mixing, stirring and the like. To this polyvinyl alcohol is added gradually to prepare the desired powder. The dry contents are further mixed other pharmaceutical acceptable excipients like 5% of talc and 23% of pigments to obtain the dry coating composition as per the present invention.

Above generated coating composition is taken in amount of 156 gms and reconstituted in pharmaceutically acceptable solvent (e.g. purified water) in amount of 290 gms and the suspension thus obtained has 35% w/w solids content and is used as liquid film coating composition to coat various substrates. The amount of coating applied will depend upon several factors, including the nature and functionality of the film coating, the substrate to be coated and the apparatus employed to apply the coating, etc.

Further above filtered coating dispersion is sprayed over round biconvex placebo tablets of 8mm size are taken in an amount of 4Kg in batches on 18 inch coating pan having machine MAC/18 having model number 1600/380 with 1mm gun size of schlick spray gun. Coating parameters are taken as inlet air temperature being 55-70°C and bed temperature as 38-43°C with pump at RPM 2 - 3 RPM, pan speed between 18-24 RPM and atomizing air pressure being 2 to 2.5 bar. For the purpose of present coating tablet bed to gun distance is adjusted between 4-5 inches. Coating dispersion spray rate achieved in accordance with batch coating is about 16 gm/min. At the end of coating process required weight gain is about 2.5 to 3%.

### Example-2: Process for the Preparation of the composition of the present invention

156 grams of the dry coating composition as obtained from example 1 is dispersed in 364 grams of purified water as solvent to obtain a coating composition with a solids content of 30%w/w.

Further above filtered coating dispersion is sprayed over round biconvex placebo tablets of 9.5mm size are taken in an amount of 4Kg in batches on 18 inch coating pan having machine MAC/18 having model number 1600/380 with 1mm gun size of schlick spray gun. Coating parameters are taken as inlet air temperature being 55-70°C and bed temperature as 38-43°C with pump at RPM 2 - 2.5 RPM, pan speed between 18-24 RPM and atomizing air pressure being 2 to 2.5 bar. For the purpose of present coating tablet bed to gun distance is adjusted between 4-5 inches. Coating dispersion spray rate achieved in accordance with batch coating is about 10-15 gm/min. At the end of coating process required weight gain is about 2.5 to 3%.

### Example 3: Disintegration of the tablets coated with composition of the present invention

The following are disintegration times for the rapidly disintegrating tablet of the present invention. Disintegration times were obtained using the USP Disintegration Apparatus at 37°C water. The experimental test method and resultant disintegration times observed were performed in accordance with the USP Disintegration Test Method.

Coated Tablets are placed as 1 dosage unit in each of the six tubes of the basket of basket rack assembly. Operate the apparatus using water or the specified medium as the immersion fluid, maintained at 37 ± 2. At the end of the time limit specified in the monograph, lift the basket from the fluid, and observe the tablets to see if all of the tablets have disintegrated completely. If 1 or 2 tablets fail to disintegrate completely, repeat the test on 12 additional tablets. The requirement is met if not less than 16 of the total of 18 tablets tested are disintegrated.

**Table 1: Disintegration Times in Water at 37°C (USP Disintegration Apparatus)**

| **S.No** | **Example No.** | **Disintegration Time (in minutes)** | |
|---|---|---|---|
| | | **Core Tablet** | **Coated Tablets** |
| 1 | Example 1 | 1 to 2 | 2 to 4 |
| 2 | Example 2 | 1 to 2 | 2 to 4 |

It is noted from the above table that the coated tablets have property of rapid disintegration similar to immediate release coating.

### Example 4: Viscosity of polymers when sugar is included in solution.

100% solutions of polyvinyl alcohol with a solids content of 15%w/w, 20 % w/w, 25 %w/w, 30% w/w and 35%w/w we prepared by dispersing suitable quantity of polyvinyl alcohol in purified water at ambient temperature as per quantities in the below given table. Similarly solutions with (50% polyvinyl alcohol + 50% sugar) and (50% Hydroxy propyl methyl cellulose 6 cps+ 50% sugar) with solids content of 15%w/w, 20 % w/w, 25 %w/w, 30% w/w and 35%w/w were prepared as per below table. The viscosity of the compositions was measured using Brookfield DV-E Viscometer by selecting a suitable spindle and adjusting the speed and torque to get the reading.

**Table 2: Test for Viscosity of polymer with and without sugar**

| **Sr. No.** | | **1** | **2** | **3** | **4** | **5** |
|---|---|---|---|---|---|---|
| Solids content (%w/w) | | 15 | 20 | 25 | 30 | 35 |
| Quantity of powder (g) | (a) 100% PVA | 40 | 60 | 75 | 100 | 120 |
| | (b) 50%PVA + 50%Lactose | | | | | |
| | (c) 50%HPMC 6cps + 50%Sugar | | | | | |
| Quantity of purified water (g) | | 226.66 | 240 | 225 | 233.33 | 222.85 |
| (a) Viscosity of 100% PVA solution (cP) | | 625 | 1330 | 4481 | Very Viscous (not measurable) | Very Viscous (not measurable) |
| (b) Viscosity of 50% PVA + 50% Lactosesolution (cP) | | 28.5 | 68.2 | 231 | 945 | 1953 |
| (c)Viscosity of 50% HPMC + 50% Sugar solution (cP) | | 118 | 512 | 2359 | 10020 | Very Viscous (not measurable) |

From the above table it is noted that, increase in solids content causes an increase in viscosity, however it is surprisingly seen that inclusion of sugar in the solution significantly reduces the viscosity of the solution. It is further seen that combination of polyvinyl alcohol and sugar gives a much lower viscosity as compared to combination of hydroxyl propyl methyl cellulose and sugar.

### Example 5: Effect on viscosity when quantity of sugar in the coating composition of present invention is replaced with equivalent quantity of polymer

The coating composition as per example 1 having sugar is prepared with variable solids content of 25 %w/w, 30% w/w, 35%w/w and 40%w/w respectively. Another set of coating compositions where the quantity of the sugar is replaced by an equivalent quantity of polyvinyl alcohol, were prepared at 25 %w/w, 30% w/w, 35%w/w and 40%w/w respectively as per the below given table. The viscosity of these compositions was measured using Brookfield DV-E Viscometer by selecting a suitable spindle and adjusting the speed and torque to get the reading. It was seen that viscosity generally increases with the increase in solids content for reconstitution. It is further surprisingly found that the inclusion of sugar in the coating composition significantly decreased the viscosity and helped in easy spraying of the coating at high solids content of 35%w/w without any coating problems like gun chocking.

**Table 3:Test for Viscosity of polymer with and without sugar**

| **Sr. No.** | | **1** | **2** | **3** | **4** |
|---|---|---|---|---|---|
| Solids content (%w/w) | | 25 | 30 | 35 | 40 |
| Quantity of powder (g) | Coating composition as per example 1 | 75 | 100 | 120 | 140 |
| | Coating composition where sugar is replaced with polyvinyl alcohol | | | | |
| Quantity of purified water (g) | | 225 | 233.33 | 222.85 | 210 |
| Viscosity of coating composition as per example 1 having sugar (cP) | | 48.71 | 168 | 378.5 | 1150 |
| Viscosity of coating composition where sugar is replaced with polyvinyl alcohol (cP) | | 1200 | 1900 | Very viscous | Very viscous |

From the above table it is noted that, when polymer content in the coating compositions is increased there is sizeable increase in the viscosity.

### Example 6: Comparative analysis of the Viscosity

It was surprisingly seen that the combination of sugar and polymer causes reduction in viscosity of the coating composition even when it is reconstituted at a solids level of 35%. To check the difference in viscosity, representative examples from prior art having polyvinyl alcohol were prepared according to table 4 and reconstituted with 35% solids content in purified water. Their viscosities were compared to the viscosity of the composition as per Example 1 of the present invention. The viscosity of these compositions was measured using Brookfield DV-E Viscometer by selecting a suitable spindle and adjusting the speed and torque to get the reading. The results obtained are given in Table 4.

**Table 4: Comparative analysis of Viscosity**

| **Serial Number** | **Contents** | **Viscosity at 35% solids content (cP)** |
|---|---|---|
| Example 1 of present invention | 25% Polyvinyl alcohol, 31% lactose monohydrate, 3% glyceryl monostearate, 1% polysorbate-80, 12% triacetin, 5% talc, 23% colorants | 378.5 |
| Representative Example 1 of WO1996001874 | 45.52% polyvinyl alcohol, 20% talc, 32% titanium dioxide, 0.48% xanthan gum, and 2% soya lecithin | 3725 |
| Representative Example 1 of WO2006111981 | 44% polyvinyl alcohol, 15% talc, 10% titanium dioxide, 3% lake sunset yellow, 8% PEG 6000, and 20% self- emulsifying glyceryl monostearate | 2058 |
| Representative Example 1 of WO2006111980 | 45% polyvinyl alcohol, 10% PEG 6000, 20% self-emulsifying glyceryl monostearate, 9% titanium dioxide, 5%lake color, 10% flavouring agent and 1% sweetening agent. | 2423 |
| Representative Example 1 of WO2010132204 | 35% polyvinyl alcohol, 4% Eudragit L100-55, 12% PEG 3350, 23.88% talc, 20% titanium dioxide, 5% Blue #2 lake color and 0.12% sodium bicarbonate. | 2735 |
| Representative Example 2 of WO2010132205 | 35% polyvinyl alcohol copolymer, 27% talc, 4.5% micronized glyceryl monostearate, 6% triethyl citrate, 2.5% sodium lauryl sulphate, 20% titanium dioxide, 5% Blue #2 lake color | 655 |
| Representative Example 5 of WO2012120364 | 57% Polymer Composite Base (Composition as per example 1 of the patent comprising 41.04% PVA, 4.6626%Glyceryl Monostearate, 1.8639% Tween 80, 9.4335% Diethyl Phthalate) , 8% Polyethylene Glycol, 3% Magnesium stearate, 15% Titanium dioxide, 17% pigments. | 3275 |
| Representative Example 5 of WO2012120366 | 63% Hydrophobic Polymer Composite Base (Composition as per example 1 of the patent comprising 42.21% PVA, 2.9% Glyceryl Monostearate, 2.025% Tween 80, 8.173% Diethyl Phthalate, 7.692% Stearic acid) , 3% Calcium carbonate, 4% Magnesium stearate, 17% Titanium dioxide, 13% pigments. | 2807 |

It is seen that the viscosity of the composition as per example 1 of present invention is very less as compared to other representative examples. From the results it is clear that the combination of polyvinyl alcohol with a sugar in a specific range of ratio causes reduction in the viscosity of the coating composition. Additionally, the presence of plasticizer aids in providing the film with good strength and adhesion. The low viscosity of the coating composition in spite of it being dispersed at 35 % solids content make it ideal for obtaining greater coating output per hour, saving production time.

### Example 7: Trials undertaken on Thomas Engineering's Continuous Coating Machine

The coating composition prepared as per Example 1 having a solids content of 35% was used to coat a 1000 kg batch of placebo tablets in a 24 inch fully perforated drum containing Continuous Coating Machine by Thomas Engineering (Model: Thomas Flex CTC) equipped with 22 spray guns (Model: Schlick ABC Technology 940). 22 spray guns were divided into 2 separate Manifolds (11 Spray Guns per manifold). The tablets were continuously fed to the perforated drums via the Thomas COMPU-COAT^{®} control system. After moving through the spray zone, where a desired amount of coating was applied, tablets were moved into a waxing /cooling zone and then discharged. Coated tablets samples were collected from the discharge point of the Continuous coating machine once tablets achieved desired weight gain of 2.5 %.

The process parameters used while coating the tablets in continuous coating machine are given in Table 5. The coating process was also evaluated and the results of the same are given in Table 6.

**Table 5: Coating Process Parameters in the Continuous Coating Machine**

| **Coating Pan Details** | |
|---|---|
| Pan Diameter ( Inches) | 24 |

| **Spray Gun Details** | |
|---|---|
| Gun Type | Schlick |
| No. of Guns | 22 |
| Nozzle Diameter (mm) | 1.0 |

| **Coating Suspension Details** | |
|---|---|
| Solids Content (% w/w) | 35 |
| Solvent | Purified Water |
| Stirring Time (minutes) | 45 |

| **Coating Process Parameters** | |
|---|---|
| Desired Weight Gain (%) | 2.5 |
| Batch Size (Kg.) | 1000 |
| Inlet Temp (°C) | 59-64 |
| Bed Temp (°C) | 43-45 |
| Exhaust Temp. (°C) | 47-48 |
| Airflow (cfm) | 6700 |
| Atomizing Air Pressure (Psi) | 40-42 |
| Pattern Air Pressure (Psi) | 30-32 |
| Pan speed (rpm) | 10-12 |
| Spray Rate (g/min) | 548-756 |
| Weigh belt feed rate (kg/hr) | 544-635 |
| Environmental Equivalence Factor | 7.265-11.256 |

**Table 6: Coating Process Evaluation**

| **Parameters** | | **Observations** | |
|---|---|---|---|
| **(A) Process Feasibility** | | | |
| Ease of operation | | No gun choking observed during the coating process | |
| Sprayability | | Free-flowing, lumps free and grittiness free coating suspension | |

| **(B) Coating Defects** | | | |
|---|---|---|---|
| Sticking & Picking | | No coating defects observed | |
| Peel off | | | |
| Logo Bridging | | | |
| Roughness | | | |
| Any Other | | | |

| | **(C) Tablet Appearance** | | |
|---|---|---|---|
| Smoothness | | Smooth Tablet Surface | |
| Logo Clarity | | Good Logo Clarity | |

It was seen that the coating composition as per Example 1, at high solids level of 35% was capable of achieving color uniformity in less amount of time than in traditional auto-coaters, with smooth tablet surface, no visible coating defects and no gun blocking.

### Example 8: Illustrative composition of the present invention

**Table B: Composition of the present invention**

| **Ingredients** | **Std. Formula as % w/w** |
|---|---|
| Polyvinyl Alcohol | 34.4 |
| Glyceryl Monostearate | 4 |
| Polysorbate 80 | 1.6 |
| Diethyl Phthalate | 8 |
| Triacetin | 3 |
| HPMC | 12 |
| Lactose monohydrate | 11 |
| Colorants | 26 |
| **Total** | **100** |

Process of preparation of composition comprises mixing of 8% of Diethyl phthalate, 4% of glyceryl monostearate, 1.6% of polysorbate-80 and 11% of lactose monohydrate to obtain homogenous dispersion. The homogenization of dispersion may be achieved by heating, mixing, stirring and the like. To this 34.4% polyvinyl alcohol and 12% of HPMC (6cps) is added gradually to prepare the desired powder. The dry contents are further mixed with other pharmaceutical excipients like 26% of colorants to obtain the dry coating composition as per the present invention. The dry film coating composition obtained from above was reconstituted in water at a solids level of 35% w/w.

### Example 9: Illustrative composition of the present invention

**Table C: Composition of the present invention**

| **Ingredients** | **Std. Formula as % w/w** |
|---|---|
| Polyvinyl Alcohol | 28.5 |
| Glyceryl Monostearate | 3.5 |
| Polysorbate 80 | 1.5 |
| Triacetin | 11.5 |
| HPMC | 20 |
| Lactose monohydrate | 10 |
| Colorants | 25 |
| **Total** | **100** |

Process of preparation of composition comprises mixing of 11.5% of triacetin, 3.5% of glyceryl monostearate, 1.5% of polysorbate-80 and 10% of lactose monohydrate to obtain homogenous dispersion. The homogenization of dispersion may be achieved by heating, mixing, stirring and the like. To this 28.5% polyvinyl alcohol and 20% of HPMC (6cps) is added gradually to prepare the desired powder. The dry contents are further mixed with other pharmaceutical excipients like 25% of colorants to obtain the dry coating composition as per the present invention. The dry film coating composition obtained from above was reconstituted in water at a solids level of 35% w/w.

### Example 10: Illustrative composition of the present invention

**Table D: Composition of the present invention**

| **Ingredients** | **Std. Formula as % w/w** |
|---|---|
| Polyvinyl Alcohol | 28.5 |
| Glyceryl Monostearate | 3 |
| Polysorbate 80 | 1.5 |
| Triacetin | 11 |
| HPMC | 17 |
| Lactose monohydrate | 10 |
| Talc | 4 |
| Colorants | 25 |
| **Total** | **100** |

Process of preparation of composition comprises mixing of 11% of triacetin, 3% of glyceryl monostearate, 1.5% of polysorbate-80 and 10% of lactose monohydrate to obtain homogenous dispersion. The homogenization of dispersion may be achieved by heating, mixing, stirring and the like. To this 28.5% polyvinyl alcohol and 17% of HPMC (6cps) is added gradually to prepare the desired powder. The dry contents are further mixed with other pharmaceutical excipients like 4% of talc and 25% of colorants to obtain the dry coating composition as per the present invention. The dry film coating composition obtained from above was reconstituted in water at a solids level of 35% w/w.

### Example 11: Illustrative composition of the present invention

**Table E: Composition of the present invention**

| **Ingredients** | **Std. Formula as % w/w** |
|---|---|
| Polyvinyl Alcohol | 34.4 |
| Glyceryl Monostearate | 4 |
| Polysorbate 80 | 1.6 |
| Diethyl Phthalate | 12 |
| Lactose monohydrate | 28 |
| TiO₂ | 20 |
| **Total** | **100** |

Process of preparation of composition comprises mixing of 12% of diethyl phthalate, 4% of glyceryl monostearate, 1.6% of polysorbate-80 and 28% of lactose monohydrate to obtain homogenous dispersion. The homogenization of dispersion may be achieved by heating, mixing, stirring and the like. To this 34.4% polyvinyl alcohol is added gradually to prepare the desired powder. The dry contents are further mixed with other pharmaceutical excipients like 20% of titanium dioxide to obtain the dry coating composition as per the present invention. The dry film coating composition obtained from above was reconstituted in water at a solids level of 35% w/w.

### Example 12: Illustrative composition of the present invention

**Table F: Composition of the present invention**

| **Ingredients** | **Std. Formula as % w/w** |
|---|---|
| Polyvinyl Alcohol | 28.5 |
| Glyceryl Monostearate | 3 |
| Polysorbate 80 | 1.5 |
| Triacetin | 11 |
| Talc | 4 |
| Lactose monohydrate | 27 |
| TiO₂ | 25 |
| **Total** | **100** |

Process of preparation of composition comprises mixing of 11% of triacetin, 3% of glyceryl monostearate, 1.5% of polysorbate-80 and 27% of lactose monohydrate to obtain homogenous dispersion. The homogenization of dispersion may be achieved by heating, mixing, stirring and the like. To this 28.5% polyvinyl alcohol is added gradually to prepare the desired powder. The dry contents are further mixed with other pharmaceutical excipients like 4% of talc and 25% of titanium dioxide to obtain the dry coating composition as per the present invention. The dry film coating composition obtained from above was reconstituted in water at a solids level of 35% w/w.

### Example 13: Illustrative composition of the present invention

**Table G: Composition of the present invention**

| **Ingredients** | **Std. Formula as % w/w** |
|---|---|
| Polyvinyl Alcohol | 25 |
| Glyceryl Monostearate | 3 |
| Polysorbate 80 | 1 |
| Diethyl Phthalate | 10 |
| HPMC | 21 |
| Talc | 7 |
| Lactose monohydrate | 10 |
| TiO₂ | 23 |
| **Total** | **100** |

Process of preparation of composition comprises mixing of 10% of diethyl phthalate, 3% of glyceryl monostearate, 1% of polysorbate-80 and 10% of lactose monohydrate to obtain homogenous dispersion. The homogenization of dispersion may be achieved by heating, mixing, stirring and the like. To this 25% polyvinyl alcohol and 21% of HPMC (6cps) is added gradually to prepare the desired powder. The dry contents are further mixed with other pharmaceutical excipients like 7% of talc and 23% of titanium dioxide to obtain the dry coating composition as per the present invention. The dry film coating composition obtained from above was reconstituted in water at a solids level of 35% w/w.

### Example 14: Illustrative composition of the present invention

**Table H: Composition of the present invention**

| **Ingredients** | **Std. Formula as % w/w** |
|---|---|
| Polyvinyl Alcohol | 24 |
| Lactose monohydrate | 31 |
| Triacetin | 15 |
| Talc | 5 |
| TiO₂ | 25 |
| **Total** | **100** |

Process of preparation of composition comprises mixing of 15% of triacetin and 31% of lactose monohydrate to obtain homogenous dispersion. The homogenization of dispersion may be achieved by heating, mixing, stirring and the like. To this 24% polyvinyl alcohol is added gradually to prepare the desired powder. The dry contents are further mixed with other pharmaceutical excipients like 5% of talc and 25% of titanium dioxide to obtain the dry coating composition as per the present invention. The dry film coating composition obtained from above was reconstituted in water at a solids level of 35% w/w.

### Example 15: Illustrative composition of the present invention

**Table I: Composition of the present invention**

| **Ingredients** | **Std. Formula as % w/w** |
|---|---|
| Polyvinyl Alcohol | 22 |
| Lactose monohydrate | 35 |
| Triacetin | 12 |
| Talc | 5 |
| TiO₂ | 26 |
| **Total** | **100** |

Process of preparation of composition comprises mixing of 12% of triacetin and 35% of lactose monohydrate to obtain homogenous dispersion. The homogenization of dispersion may be achieved by heating, mixing, stirring and the like. To this 22% polyvinyl alcohol is added gradually to prepare the desired powder. The dry contents are further mixed with other pharmaceutical excipients like 5% of talc and 26% of titanium dioxide to obtain the dry coating composition as per the present invention. The dry film coating composition obtained from above was reconstituted in water at a solids level of 35% w/w.

### Example 16: Illustrative composition of the present invention

**Table J: Composition of the present invention**

| **Ingredients** | **Std. Formula as % w/w** |
|---|---|
| Polyvinyl Alcohol | 28 |
| Diethyl Phthalate | 15 |
| Lactose monohydrate | 31 |
| Colorants | 26 |
| **Total** | **100** |

Process of preparation of composition comprises mixing of 15% of diethyl phthalate and 31% of lactose monohydrate to obtain homogenous dispersion. The homogenization of dispersion may be achieved by heating, mixing, stirring and the like. To this 28% polyvinyl alcohol is added gradually to prepare the desired powder. The dry contents are further mixed with other pharmaceutical excipients like 26% of colorants to obtain the dry coating composition as per the present invention. The dry film coating composition obtained from above was reconstituted in water at a solids level of 35% w/w.

### Example 17: Illustrative composition of the present invention

**Table K: Composition of the present invention**

| **Ingredients** | **Std. Formula as % w/w** |
|---|---|
| Polyvinyl Alcohol | 40 |
| Glyceryl Monostearate | 4.5 |
| Polysorbate 80 | 2 |
| Triacetin | 13.5 |
| Talc | 5 |
| Lactose monohydrate | 35 |
| **Total** | **100** |

Process of preparation of composition comprises mixing of 13.5% of triacetin, 4.5% of glyceryl monostearate, 2% of polysorbate-80 and 35% of lactose monohydrate to obtain homogenous dispersion. The homogenization of dispersion may be achieved by heating, mixing, stirring and the like. To this 40% polyvinyl alcohol is added gradually to prepare the desired powder. The dry contents are further mixed with other pharmaceutical excipients like 5% of talc to obtain the dry coating composition as per the present invention. The dry film coating composition obtained from above was reconstituted in water at a solids level of 35% w/w.

## Claims

1. A dry immediate release film coating composition comprising:
- a polymer which is polyvinyl alcohol, wherein the polyvinyl alcohol is present in the range from 20 % w/w to 50 % w/w by total weight of the composition;
- a sugar present in the range from 5% w/w to 40% w/w by total weight of the composition; and
- a plasticizer present in the range from 5 % w/w to 20 % w/w by total weight of the composition;
wherein the ratio of polyvinyl alcohol to sugar is in the range from 0.5:1 to 3:1.

2. The dry immediate release film coating composition according to claim 1 wherein, the polyvinyl alcohol is present in the range from 24 % w/w to 42 % w/w by total weight of the composition.

3. The dry immediate release film coating composition according to claim 1 wherein, the sugar comprises glucose, galactose, mannose, fructose, sucrose, lactose monohydrate, lactulose, maltose, trehalose or isomaltose.

4. The dry immediate release film coating composition according to claim 1 and 3 wherein, the sugar is present in the range from 8% w/w to 35% w/w by total weight of the composition.

5. The dry immediate release film coating composition according to claim 1 wherein, the plasticizer comprises triacetin, di-butyl sebacate (DBS), di-ethyl phthalate (DEP), dibutyl phthalate(DBP), ethyl phthalate, methyl phthalate, dipropyl phthalate, glycerine, triethyl citrate (TEC), or combinations thereof.

6. The dry immediate release film coating composition according to claim 1 wherein the composition comprises other pharmaceutical excipients, the other pharmaceutical excipients comprising a plasticizer, a waxy component, a wetting agent, an additional polymer, a colorant, a glidant or combinations thereof.

7. The dry immediate release film coating composition according to claim 6 wherein the waxy component comprises glycerol mono-stearate, glyceryl monooleate, glyceryl behenate, glyceryl palmito stearate, magnesium stearate, sorbitan esters, stearic acid, palmitic acid, polyoxyethylene alkyl ethers, lauroyl polyoxyl glycerides and stearoyl polyoxyl glycerides, ceto stearyl alcohol, cetyl alcohol, cerosin, propylene glycol monostearate, sorbitan tri stearate, sodium stearyl fumarate, stearyl alcohol, hydrogenated vegetable oil, carnauba wax, microcrystalline wax, lanolin, myristic acid, petrol atum/lanolin alcohols, or derivatives of stearate.

8. The dry immediate release film coating composition according to claim 6 wherein, the waxy component is present in the range from 0.1% w/w to 10 % w/w of the composition.

9. The dry immediate release film coating composition according to claim 6 wherein, the wetting agent comprises polysorbate-80, ammonium lauryl sulfate, or sodium lauryl sulfate.

10. The dry film coating composition according to claim 6 wherein, the wetting agent is present in the range from 0.1% w/w to 4 % w/w of the total weight of the composition.

11. The dry immediate release film coating composition according to claim 6 wherein, the additional polymer comprises cellulosic polymers, acrylic polymers, synthetic polymers, methacrylic acid copolymers or derivatives thereof, and the additional polymer is present in the range from 0.1 % w/w to 30 % w/w by total weight of the composition.

12. The dry immediate release film coating composition according to claim 6 wherein, the colorant comprises FD&C colors, lake colors or mica base pearlescent pigments, and the colorant is present in the range from 18 % w/w to 28 % w/w by total weight of the composition.

13. The dry immediate release film coating composition according to claim 6 wherein, the glidant comprises talc, magnesium stearate, silica, starch, magnesium silicate or magnesium trisilicate and the glidant is present in the range from 0.1% w/w to 10% w/w of the composition.

14. A liquid sprayable immediate release film coating composition comprising the dry film coating composition of claim 1, and a solvent comprising purified water, ethanol, methanol, isopropyl alcohol or combinations thereof,

15. A process for preparing the dry immediate release film coating composition as defined in any of claims 1-6, comprising steps of:
*a*. combining the plasticizer, sugar and optionally wetting agent, waxy component to obtain a homogenous dispersion,
b. adding the polyvinyl alcohol to the dispersion to obtain a dry powder, and
c. optionally adding additional polymer, glidant and colorants.

## Patentansprüche

1. Eine trockene Filmbeschichtungszusammensetzung mit sofortiger Freisetzung, umfassend:
- ein Polymer, welches Polyvinylalkohol ist, wobei der Polyvinylalkohol im Bereich von 20 Gew.-% bis 50 Gew.-% bezogen auf das Gesamtgewicht der Zusammensetzung vorhanden ist;
- einen im Bereich von 5 Gew.-% bis 40 Gew.-% bezogen auf das Gesamtgewicht der Zusammensetzung vorhandenen Zucker; und
- einen im Bereich von 5 Gew.-% bis 20 Gew.-% bezogen auf das Gesamtgewicht der Zusammensetzung vorhandenen Weichmacher;
wobei das Verhältnis vom Polyvinylalkohol zum Zucker im Bereich von 0,5:1 bis 3:1 liegt.

2. Die trockene Filmbeschichtungszusammensetzung mit sofortiger Freisetzung nach Anspruch 1, wobei der Polyvinylalkohol im Bereich von 24 Gew.-% bis 42 Gew.-% bezogen auf das Gesamtgewicht der Zusammensetzung vorhanden ist.

3. Die trockene Filmbeschichtungszusammensetzung mit sofortiger Freisetzung nach Anspruch 1, wobei der Zucker Glucose, Galactose, Mannose, Fructose, Saccharose, Lactosemonohydrat, Lactulose, Maltose, Trehalose und Isomaltose umfasst.

4. Die trockene Filmbeschichtungszusammensetzung mit sofortiger Freisetzung nach Anspruch 1 und 3, wobei der Zucker im Bereich von 8 Gew.-% bis 35 Gew.-% bezogen auf das Gesamtgewicht der Zusammensetzung vorhanden ist.

5. Die trockene Filmbeschichtungszusammensetzung mit sofortiger Freisetzung nach Anspruch 1, wobei der Weichmacher Triacetin, Dibutylsebacat (DBS), Diethylphthalat (DEP), Dibutylphthalat (DBP), Ethylphthalat, Methylphthalat, Dipropylphthalat, Glycerin, Triethylcitrat (TEC) oder Kombinationen davon umfasst.

6. Die trockene Filmbeschichtungszusammensetzung mit sofortiger Freisetzung nach Anspruch 1, wobei die Zusammensetzung andere pharmazeutische Hilfsstoffe umfasst, wobei die anderen pharmazeutische Hilfsstoffe einen Weichmacher, eine wachsartige Komponente, ein Benetzungsmittel, ein zusätzliches Polymer, ein Färbemittel, ein Fließregulierungsmittel oder Kombinationen davon umfassen.

7. Die trockene Filmbeschichtungszusammensetzung mit sofortiger Freisetzung nach Anspruch 6, wobei die wachsartige Komponente Glycerolmonostearat, Glycerylmonooleat, Glycerylbehenat, Glycerylpalmitostearat, Magnesiumstearat, Sorbitanester, Stearinsäure, Palmitinsäure, Polyoxyethylenalkylether, Lauroyl-Polyoxylglyceride und Stearoyl-Polyoxylglyceride, Cetostearylalkohol, Cetylalkohol, Cerosin, Propylenglycolmonostearat, Sorbitantristearat, Natriumstearylfumarat, Stearylalkohol, hydriertes Pflanzenöl, Carnaubawachs, mikrokristallines Wachs, Lanolin, Myristinsäure, Petrolatum-/Lanolin-Alkohole, oder Derivate von Stearat umfasst.

8. Die trockene Filmbeschichtungszusammensetzung mit sofortiger Freisetzung nach Anspruch 6, wobei die wachsartige Komponente im Bereich von 0,1 Gew.-% bis 10 Gew.-% der Zusammensetzung vorhanden ist.

9. Die trockene Filmbeschichtungszusammensetzung mit sofortiger Freisetzung nach Anspruch 6, wobei das Benetzungsmittel Polysorbat-80, Ammoniumlaurylsulfat, oder Natriumlaurylsulfat umfasst.

10. Die trockene Filmbeschichtungszusammensetzung nach Anspruch 6, wobei das Benetzungsmittel im Bereich von 0,1 Gew.-% bis 4 Gew.-% bezogen auf das Gesamtgewicht der Zusammensetzung vorhanden ist.

11. Die trockene Filmbeschichtungszusammensetzung mit sofortiger Freisetzung nach Anspruch 6, wobei das zusätzliche Polymer Cellulosepolymere, Acrylpolymere, synthetische Polymere, Methacrylsäurecopolymere oder Derivate davon umfasst, und das zusätzliche Polymer im Bereich von 0,1 Gew.-% bis 30 Gew.-% bezogen auf das Gesamtgewicht der Zusammensetzung vorhanden ist.

12. Die trockene Filmbeschichtungszusammensetzung mit sofortiger Freisetzung nach Anspruch 6, wobei das Färbemittel FD & C-Farben, Lackfarben oder Perlglanzpigmente auf Glimmerbasis umfasst, und das Färbemittel im Bereich von 18 Gew.-% bis 28 Gew.-% bezogen auf das Gesamtgewicht der Zusammensetzung vorhanden ist.

13. Die trockene Filmbeschichtungszusammensetzung mit sofortiger Freisetzung nach Anspruch 6, wobei das Fließregulierungsmittel Talk, Magnesiumstearat, Siliciumdioxid, Stärke, Magnesiumsilikat und Magnesiumtrisilikat umfasst, und das Fließregulierungsmittel im Bereich von 0,1 Gew.-% bis 10 Gew.-% der Zusammensetzung vorhanden ist.

14. Eine flüssige sprühbare Filmbeschichtungszusammensetzung mit sofortiger Freisetzung umfassend die trockene Filmbeschichtungszusammensetzung von Anspruch 1, und ein Lösungsmittel umfassend gereinigtes Wasser, Ethanol, Methanol, Isopropylalkohol oder Kombinationen davon.

15. Ein Verfahren zur Herstellung der trockenen Filmbeschichtungszusammensetzung mit sofortiger Freisetzung wie in einem der Ansprüche 1 bis 6 definiert, umfassend die folgenden Schritte:
a. Kombinieren des Weichmachers, des Zuckers und gegebenenfalls des Benetzungsmittels, der wachsartigen Komponente, um eine homogene Dispersion zu erhalten,
b. Hinzufügen des Polyvinylalkohols zu der Dispersion, um trockenes Pulver zu erhalten, und
c. gegebenenfalls Zugabe von zusätzlichem Polymer, Fließregulierungsmittel und Farbstoffen.

## Revendications

1. Une composition de pelliculage sèche à libération immédiate comprenant :
- un polymère qui est l'alcool polyvinylique, dans laquelle l'alcool polyvinylique est présent dans la plage allant de 20 % en poids/poids et 50 % en poids/poids par rapport au poids total de la composition ;
- un sucre présent dans la plage allant de 5 % en poids/poids à 40 % en poids/poids par rapport au poids total de la composition ; et
- un plastifiant présent dans la plage allant de 5 % en poids/poids à 20 % en poids/poids par rapport au poids total de la composition ;
dans laquelle le rapport de l'alcool polyvinylique au sucre est dans la plage allant de 0,5: 1 à 3:1.

2. La composition de pelliculage sèche à libération immédiate selon la revendication 1, dans laquelle l'alcool polyvinylique est présent dans la plage allant de 24 % en poids/poids à 42 % en poids/poids par rapport au poids total de la composition.

3. La composition de pelliculage sèche à libération immédiate selon la revendication 1, dans laquelle le sucre comprend du glucose, du galactose, du mannose, du fructose, du saccharose, du lactose monohydraté, du lactulose, du maltose, du tréhalose et de l'isomaltose.

4. La composition de pelliculage sèche à libération immédiate selon la revendication 1 et 3, dans laquelle le sucre est présent dans la plage allant de 8% en poids/poids à 35% en poids/poids par rapport au poids total de la composition.

5. La composition de pelliculage sèche à libération immédiate selon la revendication 1, dans laquelle le plastifiant comprend de la triacétine, du sébacate de dibutyle (DBS), du phtalate de diéthyle (DEP), du phtalate de dibutyle (DBP), du phtalate d'éthyle, du phtalate de méthyle, du phtalate de dipropyle, de la glycérine, du citrate de triéthyle (TEC) ou des combinaisons de ceux-ci.

6. La composition de pelliculage sèche à libération immédiate selon la revendication 1, dans laquelle la composition comprend d'autres excipients pharmaceutiques, les autres excipients pharmaceutiques comprennent un plastifiant, un composant cireux, un agent mouillant, un polymère supplémentaire, un colorant, un agent de glissement ou des combinaisons de ceux-ci.

7. La composition de pelliculage sèche à libération immédiate selon la revendication 6, dans laquelle le composant cireux comprend du monostéarate de glycérol, du monooléate de glycéryle, du béhénate de glycéryle, du palmito-stéarate de glycéryle, du stéarate de magnésium, des esters de sorbitane, de l'acide stéarique, de l'acide palmitique, des éthers alkyliques de polyoxyéthylène, des lauroyl-polyoxyle glycérides et des stéaroyl-polyoxyle glycérides, de l'alcool céto-stéarylique, de l'alcool cétylique, de la cérosine, du monostéarate de propylène glycol, du tristéarate de sorbitane, du fumarate stéarylique de sodium, de l'alcool stéarylique, de l'huile végétale hydrogénée, de la cire de carnauba, de la cire microcristalline, de la lanoline, de l'acide myristique, des alcools d'essence de pétrole/lanoline, ou des dérivés de stéarate.

8. La composition de pelliculage sèche à libération immédiate selon la revendication 6, dans laquelle le composant cireux est présent dans la plage allant de 0,1% en poids/poids à 10 % en poids/poids de la composition.

9. La composition de pelliculage sèche à libération immédiate selon la revendication 6, dans laquelle l'agent mouillant comprend du polysorbate-80, du laurylsulfate d'ammonium, ou du laurylsulfate de sodium.

10. La composition de pelliculage sèche selon la revendication 6, dans laquelle l'agent mouillant est présent dans la plage allant de 0,1 % en poids/poids à 4 % en poids/poids par rapport au poids total de la composition.

11. La composition de pelliculage sèche à libération immédiate selon la revendication 6, dans laquelle le polymère supplémentaire comprend des polymères cellulosiques, des polymères acryliques, des polymères synthétiques, des copolymères d'acide méthacrylique et des dérivés de ceux-ci, et le polymère supplémentaire est présent dans la plage allant de 0,1 % en poids/poids à 30 % en poids/poids par rapport au poids total de la composition.

12. La composition de pelliculage sèche à libération immédiate selon la revendication 6, dans laquelle le colorant comprend des couleurs FD & C, des couleurs laquées ou des pigments nacrés à base de mica, et le colorant est présent dans la plage allant de 18 % en poids/poids à 28 % en poids/poids par rapport au poids total de la composition.

13. La composition de pelliculage sèche à libération immédiate selon la revendication 6, dans laquelle l'agent de glissement comprend du talc, du stéarate de magnésium, de la silice, de l'amidon, du silicate de magnésium et du trisilicate de magnésium et l'agent de glissement est présent dans la plage allant de 0,1 % en poids/poids à 10 % en poids/poids de la composition.

14. Une composition de pelliculage liquide à libération immédiate pulvérisable comprenant la composition de pelliculage sèche de la revendication 1, et un solvant comprenant de l'eau purifiée, de l'éthanol, du méthanol, de l'alcool isopropylique ou des combinaisons de ceux-ci.

15. Un procédé de préparation de la composition de pelliculage sèche à libération immédiate telle que définie dans l'une quelconque des revendications 1 à 6, comprenant les étapes consistant à :
*a*. combiner le plastifiant, le sucre et éventuellement l'agent mouillant, le composant cireux, pour obtenir une dispersion homogène,
b. ajouter l'alcool polyvinylique à la dispersion pour obtenir une poudre sèche, et
c. ajouter éventuellement du polymère, du glissant et des colorants supplémentaires.
